# EUROPEAN PATENT APPLICATION

(11) **EP 2 181 713 A1**
(43) Date of publication of application: **05.05.2010**
(21) Application number: 09174160.3
(22) Date of filing: 27.10.2009
(51) Int. Cl.: A61K 38/17, A61P 31/04, A61P 29/00, A61P 35/00

(54) **Pharmaceutical compositions for prevention or treatment of Lewis Y antigen related diseases and kits for detecting Lewis Y antigen**

(30) Priority: 30.10.2008 US 261736
(71) Applicant: Wu, Hua-Lin, Tainan 701 (TW); Shi, Guey-Yueh, Tainan 701 (TW)
(72) Inventor: Wu, Hua-Lin, Tainan 701 (TW); Shi, Guey-Yueh, Tainan 701 (TW)
(74) Representative: Viering, Jentschura & Partner

(57) **Abstract**

The present invention discloses use of a composition comprising N-terminal lectin-like domain of thrombomodulin (TMD1) or analogues thereof in the preparation of a medicament for the treatment or prevention of a disease resulting from Lewis Y antigen over-expression; and a kit and method of detecting cancer cells, Gram-negative bacteria or LPS which over-express the Lewis Y antigen in a sample from a subject.

SEQUENCE LISTING

<110>  Wu, Hua-Lin
<110>  Shi, Guey-Yueh

<120>  Pharmaceutical compositions for prevention or treatment of Lewis Y
antigen related diseases and kits for detecting Lewis Y antigen

<130>  P41289

<150>  US 12/261,736
<151>  October 30, 2008

<160>  6

<170>  PatentIn version 3.4

<210>  1
<211>  187
<212>  PRT
<213>  Human

<220>
<221>  DOMAIN
<222>  (1)..(187)

<400>  1

Glu Phe Ala Pro Ala Glu Pro Gln Pro Gly Gly Ser Gln Cys Val Glu
1               5                   10                  15

His Asp Cys Phe Ala Leu Tyr Pro Gly Pro Ala Thr Phe Leu Asn Ala
            20                  25                  30

Ser Gln Ile Cys Asp Gly Leu Arg Gly His Leu Met Thr Val Arg Ser
        35                  40                  45

Ser Val Ala Ala Asp Val Ile Ser Leu Leu Leu Asn Gly Asp Gly Gly
    50                  55                  60

Val Gly Arg Arg Arg Leu Trp Ile Gly Leu Gln Leu Pro Pro Gly Cys
65                  70                  75                  80

Gly Asp Pro Lys Arg Leu Gly Pro Leu Arg Gly Phe Gln Trp Val Thr
                85                  90                  95

Gly Asp Asn Asn Thr Ser Tyr Ser Arg Trp Ala Arg Leu Asp Leu Asn
            100                 105                 110

Gly Ala Pro Leu Cys Gly Pro Leu Cys Val Ala Val Ser Ala Ala Glu
        115                 120                 125

Ala Thr Val Pro Ser Glu Pro Ile Trp Glu Glu Gln Gln Cys Glu Val
    130                 135                 140

Lys Ala Asp Gly Phe Leu Cys Glu Phe His Phe Pro Ala Tyr Leu Glu
145                 150                 155                 160

Asp Asp Asp Asp Lys Ile Glu Gln Lys Leu Ile Ser Glu Glu Asp Leu
                165                 170                 175

Asn Ser Ala Val Asp His His His His His His
            180                 185

<210>  2
<211>  29
<212>  DNA
<213>  Artificial

<220>
<223>  TMD-1 sense

<220>
<221>  misc_feature
<222>  (1)..(29)

<400>  2
ttggaattcg caggggcgag ccgcagccg                                        29

<210>  3
<211>  29
<212>  DNA
<213>  Artificial

<220>
<223>  TMD-1 antisense

<220>
<221>  misc_feature
<222>  (1)..(29)

<400>  3
aattctagat agcaggtggc tgggaagtg                                        29

<210>  4
<211>  35
<212>  DNA
<213>  Artificial

<220>
<223>  TMD-2 sense

<220>
<221>  misc_feature
<222>  (1)..(35)

<400>  4
aaggaattcg cttgggactg cagcgtggag aacgg                                 35

<210>  5
<211>  32
<212>  DNA
<213>  Artificial

<220>
<223>  TMD-3 antisense

<220>
<221>  misc_feature
<222>  (1)..(32)

<400>  5
aattctagat acgaatgcac gagccccacg gc                                    32


<210>  6
<211>  32
<212>  DNA
<213>  Artificial

<220>
<223>  TMD-2 sense and TMD2/EGF3 antisense


<220>
<221>  misc_feature
<222>  (1)..(32)

<400>  6
aattctagat actccacaca ctcgccgtcc ac                                    32

## Description

### FIELD OF THE INVENTION

The present invention relates to the N-terminal lecitn-like domain of thrombomodulin and its use for the prevention and/or the treatment of Lewis Y antigen related diseases or disorders.

### BACKGROUND OF THE INVENTION

Septic shock syndrome that results from excessive host immune responses induced by infectious organisms is a leading cause of death in hospitalized patients. Pathophysiological changes in sepsis involve the pathogen-induced uncontrolled release from immune cells, particularly monocytes and macrophages, of pro-inflammatory mediators including nitric oxide, tumor necrosis factor (TNF), and interleukins (ILs). Gram-negative bacterial infection is one of the major causes of systemic bacterial sepsis due to the lipopolysaccharide (LPS) constituent of the Gram-negative outer membrane. LPS induces a rapid increase of pro-inflammatory mediators, leading to lethal systemic tissue damage and multiple organ failure, which mimic the inflammatory responses of septic syndrome.

In mammals, membrane-bound CD 14 and Toll-like receptor-4 (TLR4)-MD-2 participate in cellular recognition of LPS. Binding of LPS to TLR4 receptor triggers the activation of the members of the mitogen-activated protein kinase (MAPK) pathway including p38, p42/p44 extra-cellular signal-regulated kinase (ERK), and c-Jun N-terminal kinase (JNK). In resting, un-stimulated cells, nuclear factor (NF)-κB, a heterodimeric complex composed of 50- and 65- kDa (p50/p65) protein subunits, is retained as an inactive complex bound to inhibitory κBα (IκBα) in the cytoplasm. While the cells are under pro-inflammatory stimulation by LPS, phosphorylation and degradation of IκBα permit NF-κB nuclear translocation and promote the expression of inflammatory genes including inducible nitric oxide synthase (iNOS), TNF-α, and others.

Thrombomodulin (TM) is a 557 amino acid type I glycosylated transmembrane protein with an NH₂-terminal lectin-like region (D1) followed by six epidermal growth factor (EGF)-like structures (D2), an O-glycosylation site-rich domain (D3), a transmembrane domain (D4), and a cytoplasmic tail domain (D5) (Weiler, H. et al. J Thromb Haemost. 2003 Jul;1(7):1515-24). D2 EGF-like structures are responsible for the anticoagulant activity of TM via the alteration of the substrate specificity of thrombin. TM domain 2 (TMD2)-thrombin complex activates anticoagulant protein C (APC), which in turn inactivates procoagulant cofactors Va and VIIIa. On the other hand, TM expression occurs in cell types including keratinocytes, polymorphonuclear neutrophils (PMNs), monocytes, and endothelial cells, indicating that TM might have biological functions in addition to anticoagulation. Different biological functions of TM may also exist. TM domain functions as an angiogenic factor and anti-inflammatory agent through protein C dependent and independent mechanisms. Recently, the anti-inflammatory activity of TM domain 1 (TMD1) was implied, since mice with a deleted TM lectin-like domain (TM^{LeD/LeD}) became more sensitive to the challenge with LPS, through the suppression of adhesion molecule expression via NFκB and MAPK signaling pathways (Conway, E.M. et al. J Exp Med. 2002 Sep 2;196(5):565-77; U.S. Pat. 73419912). Moreover, it has been demonstrated that TMD1 sequesters high-mobility group-B1 (HMGB1) protein, a late cytokine mediator of lethal endotoxemia and sepsis, by interfering the binding of HMGB1 to receptor for advanced glycation end product (RAGE) (Abeyama, K. et al. J Clin Invest. 2005 May;115(5):1267-74. Epub 2005 Apr 14). TMD1 also interferes with complement activation and protects against arthritis.

TMD1 is structurally homologous to mammalian C-type lectins that generally consist of Ca²⁺-dependent carbohydrate receptor domains. Different C-type lectins contribute to various biological functions including cell adhesion, endocytosis, and pathogen neutralization and play an important role in the innate immune system.

Lewis Y antigen overexpressed on 70% of epithelial-derived tumors is a member of a family of blood group-related difucosylated oligosaccharides (Kuemmel A et al.,Tumor Biol 2007;28:340-349). The epithelial-derived tumors include breast, pancreas, ovary, colon, gastric, and lung cancer. The high frequency of Lewis Y -expressing tumors, its high density and altered expression on the surface of tumor cells, and its relatively homogenous expression in primary and metastatic lesions have led to its selection as an antigenic target for a range of epithelial tumors, including breast cancer (Westwood JA et al. PNAS, 2005; 102(52): 19051-19056; Schlimok, G., et al. Eur. J. Cancer, 31A: 1799-1803, 1995; Tolcher, A. W., et al. J. Clin. Oncol., 17: 478-484, 1999). The antibodies recognizing Lewis Y antigen could eliminate carcinomas that express Ley (Kerrie Clarke et al. Clinical Cancer Research 6, 3621-3628, 2000; Erwin R. Boghaert et al. Clinical Cancer Research 10, 4538-4549, 2004).

### SUMMARY OF THE INVENTION

The present invention provides a use of a composition comprising N-terminal lectin-like domain of thrombomodulin (TMD1), or analogues thereof in the preparation of a medicament for treatment or prevention of a disease resulting from Lewis Y antigen over-expression.

The present invention also provides a kit of detecting Lewis Y antigen over-expressed in tumor cells, Gram-negative bacteria, or LPS comprising a labeled N-terminal lectin-like domain of thrombomodulin (TMD 1), or labeled analogues thereof.

The present invention further provides a method of detecting tumor cells, Gram-negative bacteria or LPS which over-express the Lewis Y antigen in a sample from a subject, said method comprising the steps: contacting a sample with a composition comprising a labeled N-terminal lectin-like domain of thrombomodulin (TMD 1), or labeled analogues thereof.

### BRIEF DESCRIPTION OF THE DRAWINGS

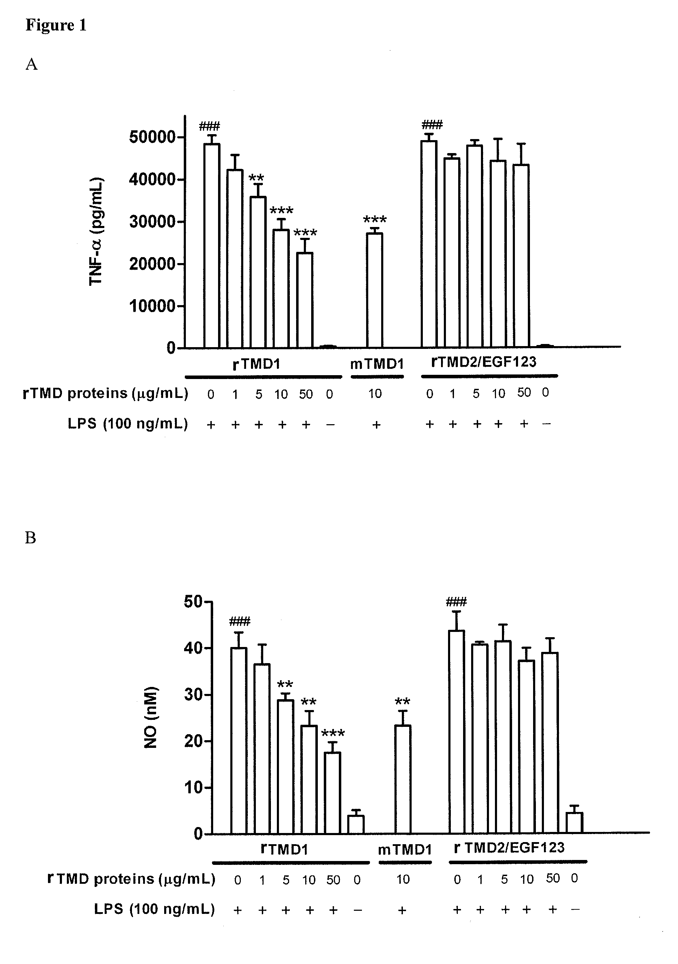

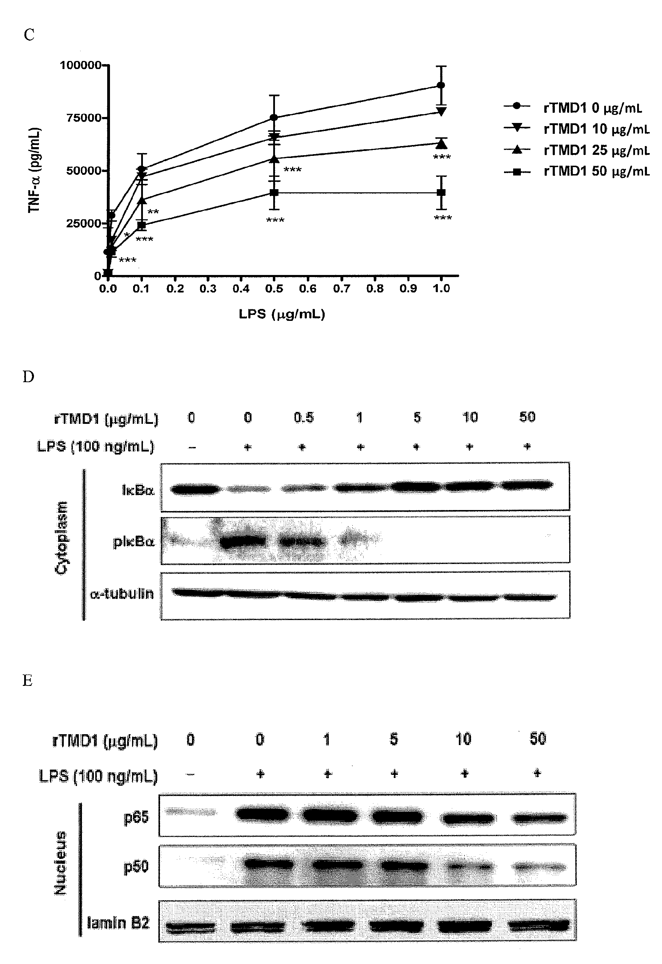

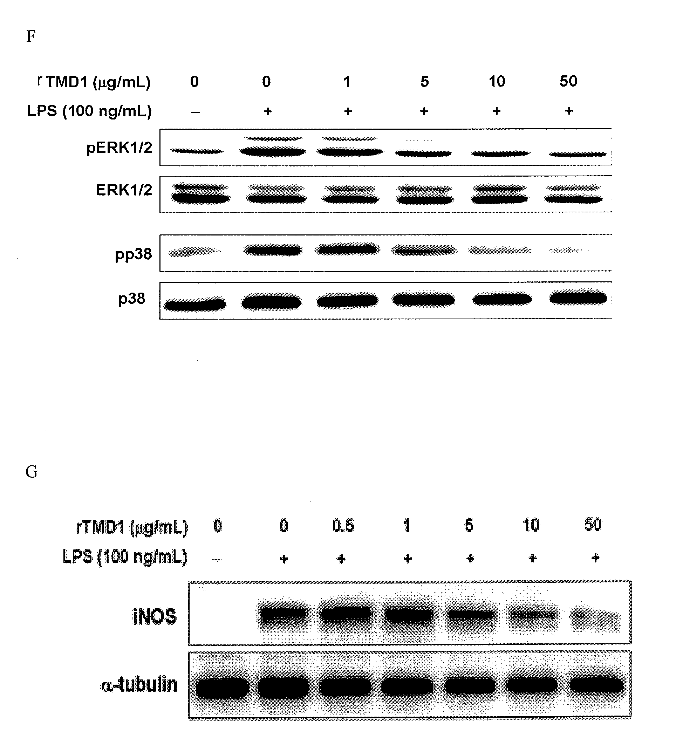

**Figure 1** shows the effects of recombinant TMD (rTMD) proteins on LPS-induced inflammatory mediator production and LPS-induced signaling. (A and B) rTMD proteins from *Pichia* and mammalian protein expression systems were pre-incubated with LPS before adding to RAW 264.7 cells. After 6 hours incubation, culture media were collected for the measurement of (A) TNF-α and (B) NO. mTMD1 represents rTMD1 from mammalian protein expression system. Values are the mean ± SD (n=3). A and B, ***P* < 0.01 and ****P* < 0.001 compared with the LPS-treated cultures. ^{###}*P* < 0.001 compared with the PBS group. (C) Various amounts of LPS were used to induce the TNF-α production in RAW264.7 cells, and the effects of rTMD1 were determined. Values are the mean ± SD (n=4). **P* < 0.05, ***P* < 0.01, and ****P* < 0.001 compared with the LPS-treated group. Western blotting was used to assay LPS-induced phosphorylation and degradation of IκBα (D), nuclear translocation of NF-κB p50 and p65 in nuclear fractions (E), ERK1/2 and p38 phosphorylation (F), and iNOS expression (G). All results are typical of those obtained in at least three independent experiments.

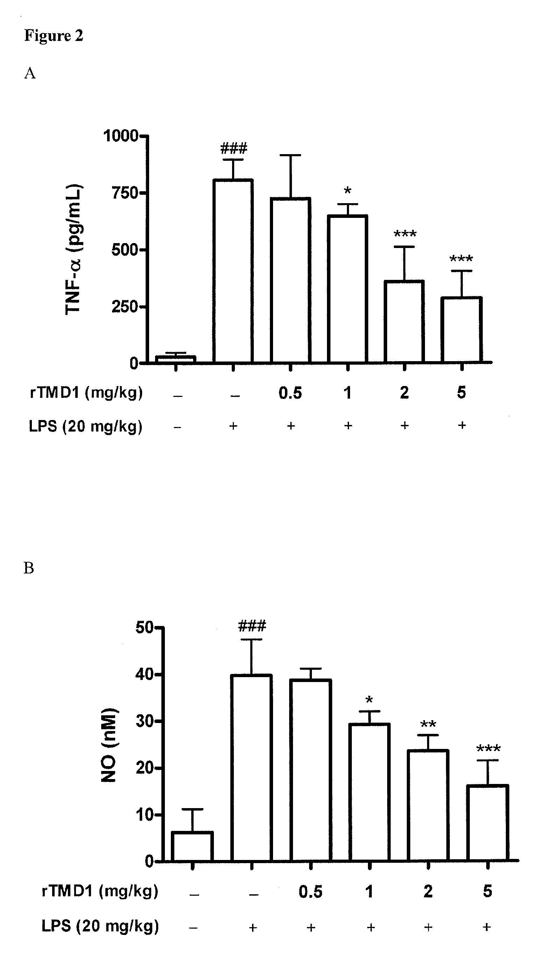

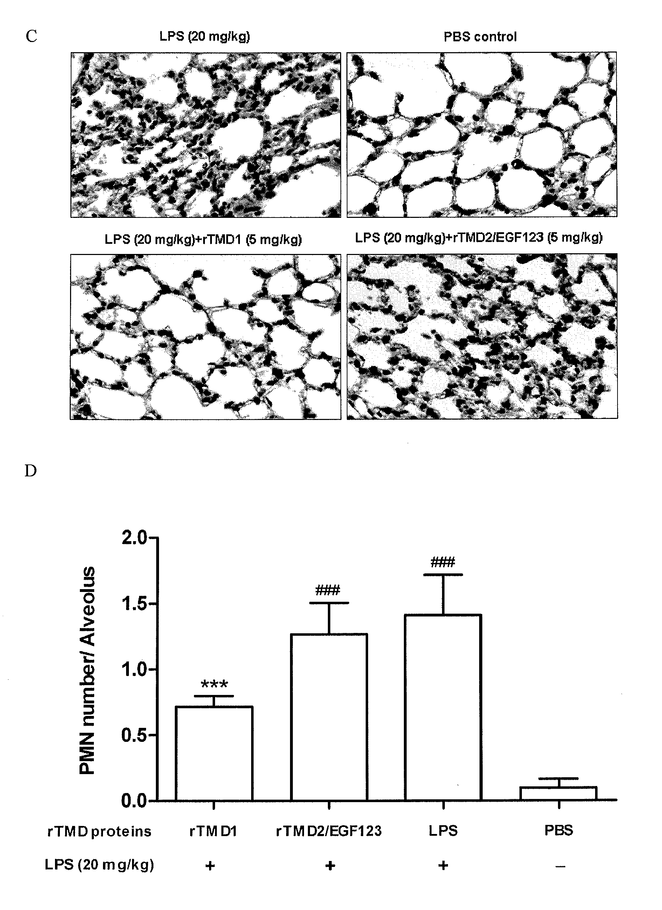

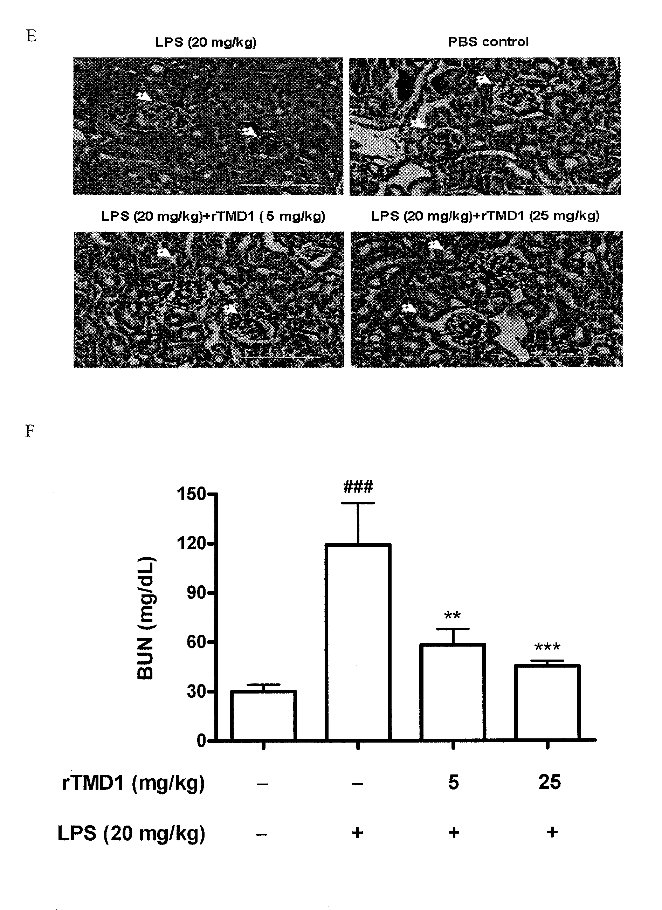

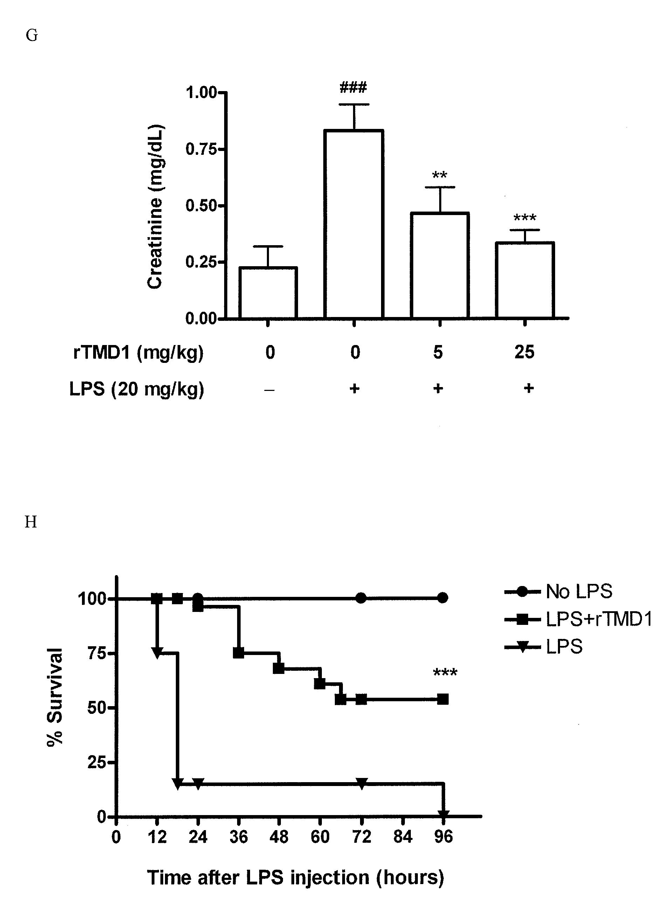

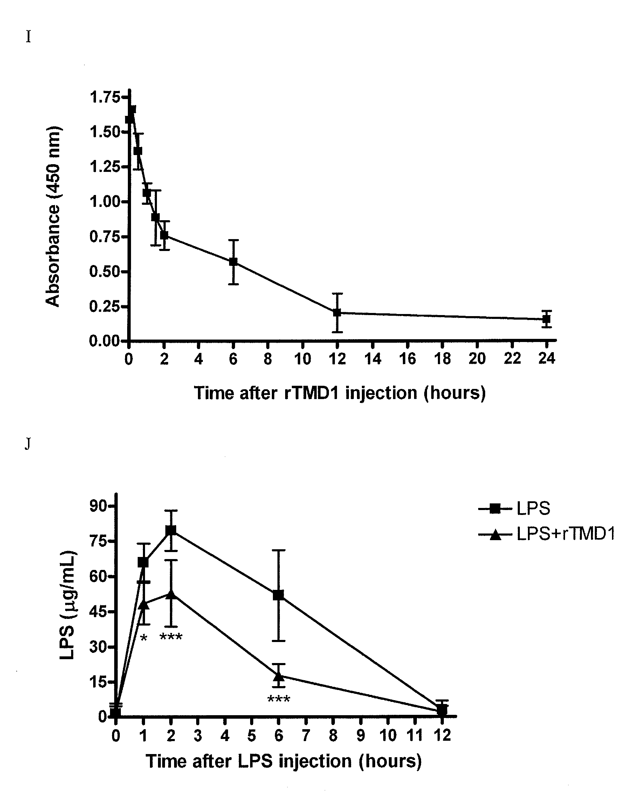

**Figure 2** shows rTMD1 reduces LPS-induced inflammatory response and lethality, attenuates LPS-induced pulmonary accumulation of PMNs and renal injury, and enhances LPS clearance in vivo. (A and B) rTMD1 was i.v.-administered before i.p. injection of LPS (20 mg/kg). Sera were collected 6 hours after administration of LPS for assay of (A) TNF-α and (B) NO production. Values are the mean ± SD (n=10). **P* < 0.05, ***P* < 0.01, ****P* < 0.001 compared with the LPS-treated group; ^{###}*P* < 0.001 compared with the PBS-treated group. (C) Representative microscopic images of hematoxylin and eosin stained sections of lung are shown. (D) The number of infiltrated PMN in each alveolus was observed by light microscope (630x magnification). The number of PMN was counted from four randomly fields per slide on each experimental mouse and normalized to fields of alveolus numbers on each slide. Values are the mean ± SD (n=10). ****P* < 0.001 compared with the LPS-treated group; ^{###}*P* < 0.001 compared with the PBS-treated group. These graphs represent the results from three independent experiments. (E, F, and G) rTMD1 (5 and 25 mg/kg) was i.v.-administrated before LPS (20 mg/kg) i.p. injection into mice. After 12 hours, mice were sacrificed and kidney tissues were removed. Kidney sections were observed by histological hematoxylin and eosin staining. Representative microscopic images are shown. (E) rTMD1 suppression of renal injury. Arrows heads indicate the site of the glomerulus (scale bars represent 50 µm). rTMD1 decreased the levels of renal injury markers BUN (F) and creatinine (G) in mice sera. For each experimental group, n=4; ^{###}*P* < 0.001 compared with the LPS-untreated control mice. ***P* < 0.01, ****P* < 0.001 compared with the LPS-treated control mice. These graphs represent the results from three independent experiments. (H) Mice received LPS (40 mg/kg) and rTMD1 (four i.v. doses of 2 mg/kg at 0, 6, 12, and 24 hours after LPS injection). Survival was determined. For each experimental group, n=20. ****P* < 0.001 compared with the LPS-treated group. (I) Clearance of rTMD1 in circulation. The half-life of rTMD1 in the circulation was determined by i.v. injection of rTMD1 (10 mg/kg) and the levels of rTMD1 in serum samples were measured by a sandwich ELISA employing anti-c-Myc and TM-H300 as capture and detection antibodies, respectively. Values are the mean ± SD (n=5). (J) Clearance of LPS in circulation without or with TMD1 treatment. LPS (20 mg/kg) was i.p.-administrated to male FVB mice without or with rTMD1 (10 mg/kg; i.v.), and serum samples were collected at various time intervals and the amount of LPS was determined by the Limulus amebocyte lysate test. Values are the mean ± SD. For each time interval group, n=5; **P* < 0.05 and ****P* < 0.001 compared with the LPS-treated mice.

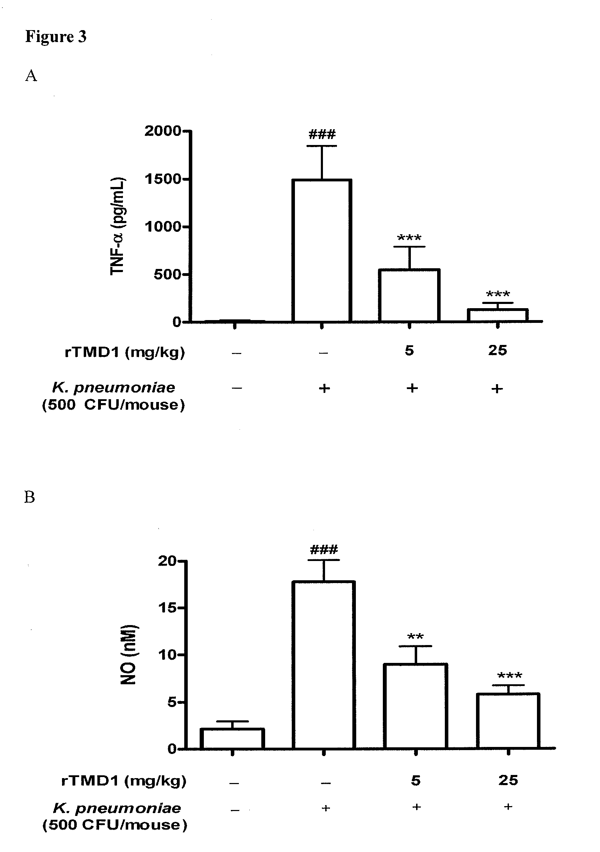

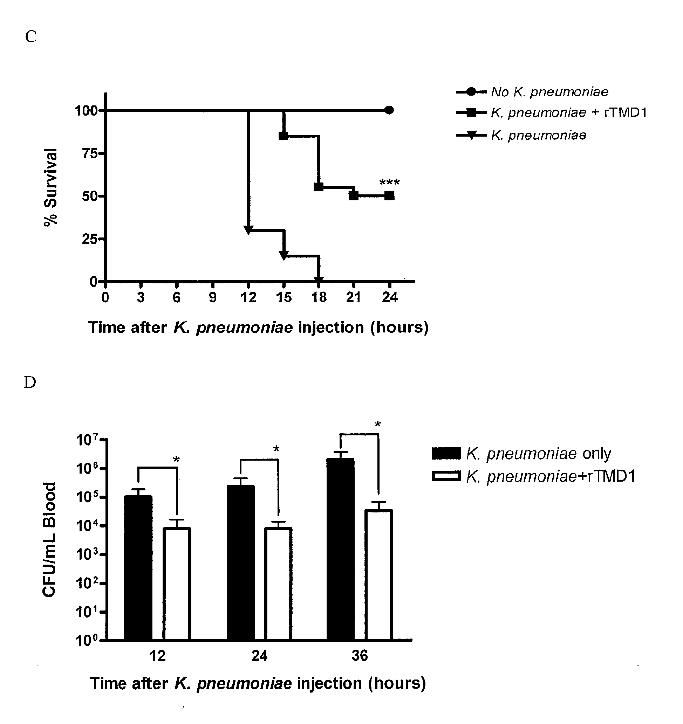

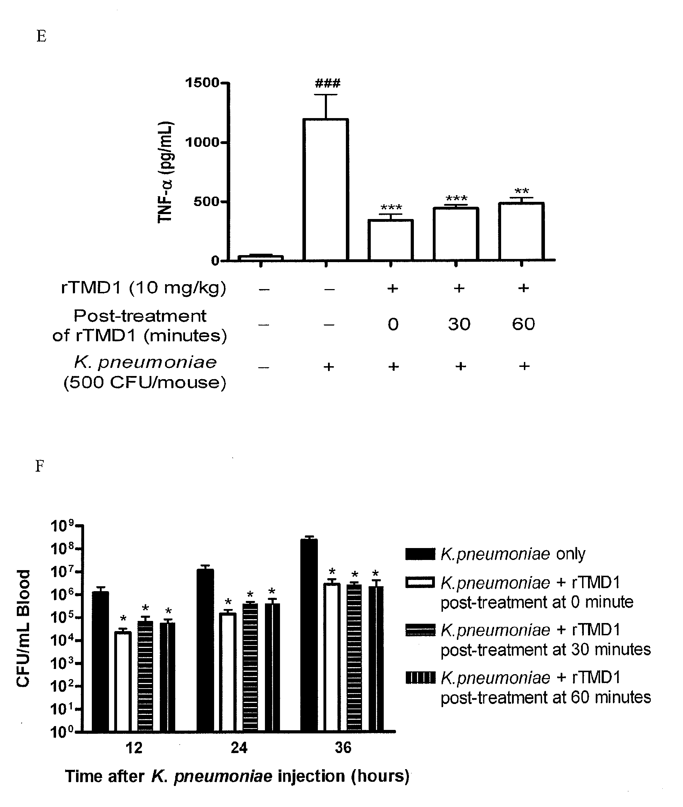

**Figure 3** shows rTMD1 reduces *K. pneumonae*-induced inflammatory response and lethality, and enhances bacterial clearance. (A and B) rTMD1 was administered before injection of *K. pneumoniae* (5×10² CFU/mouse) to mice. Sera were collected 12 hours after administration of *K. pneumoniae* for assay of (A) TNF-α and (B) NO production. Values are the mean ± SD (n=10). ***P* < 0.01 and ****P* < 0.001 compared with the *K. pneumoniae*-treated group; ^{###}*P* < 0.001 compared with the PBS-treated group. (C) rTMD1 (10 mg/kg) was administered before injection of *K. pneumoniae* (5×10³ CFU/mouse) to mice. Survival was determined. For each experimental group, n=20. ****P* < 0.001 compared with the *K. pneumoniae*-treated group. These graphs represent the results from three independent experiments. (D) rTMD1 enhanced *K. pneumoniae* clearance in circulation. FVB mice were i.p.-injected *K. pneumoniae* (5×10² CFU/mouse) without or with rTMD1 (10 mg/kg; i.v.). The blood samples from each group were collected at various time intervals and assayed for viable bacterial CFU counts. Values are the mean ± SD. For each time interval group, n=5; **P* < 0.05 compared with the *K. pneumoniae*-treated mice. (E) (F) rTMD1 (10 mg/kg) was administrated immediately, 30, or 60 minutes after injection of *K. pneumoniae* (5×10² CFU/mouse) to mice. The serum level of TNF-α (E) and *K. pneumoniae* clearance (F) were determined. Values are the mean ± SD. For each time interval group, n=8-10; **P* < 0.05 and ****P* < 0.001 compared with the *K. pheumoniae*-treated mice on each time interval group. ^{###}*P* < 0.001 compared with the PBS-treated group. Similar results were obtained in two independent experiments.

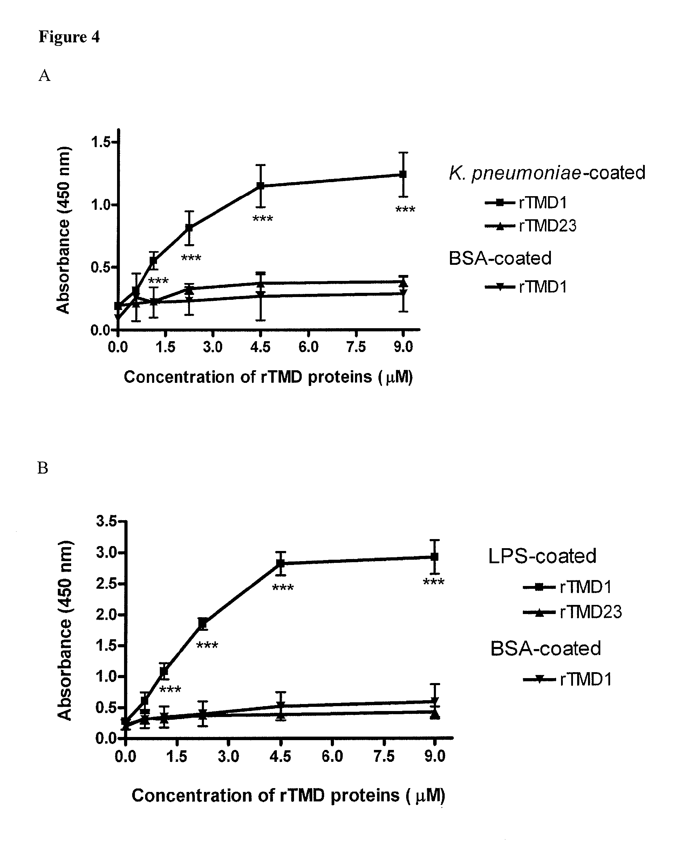

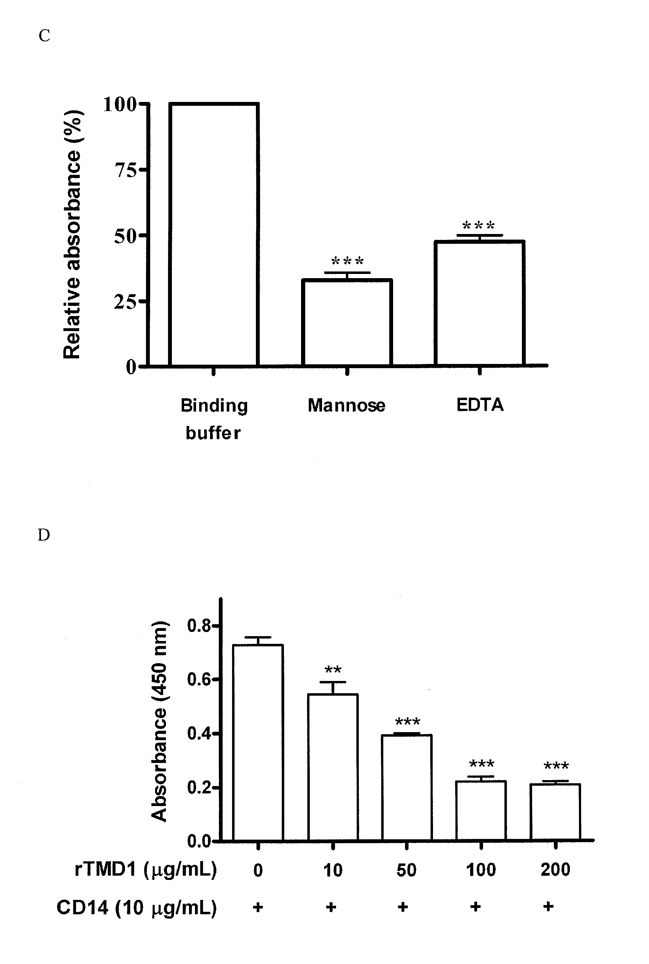

**Figure 4** shows the binding of rTMD proteins with *K. pneumoniae* and LPS, and the blocking effect of rTMD1 on the binding of CD14 to LPS. (A) *K. pneumoniae* or BSA. (B) *E. coli* O111:B4 LPS or BSA. (A and B) *K. pneumoniae*, LPS, or BSA was coated onto wells. Equi-molar amounts of rTMD proteins were added to each well. The binding of rTMD proteins was detected. Values are the mean ± SD (n=6). ****P* < 0.001 compared with the rTMD23-added group and BSA-coated group. (C) LPS was coated onto wells and incubated with rTMD1 (50 µg/mL) in binding buffer containing CaCl₂ in the absence and presence of 0.2 M mannose or 5 mM EDTA. The results are expressed as the percentage of relative absorbance normalized with binding buffer group (100%). Values are the mean ± SD (n=6). ****P* < 0.001 compared with the binding buffer group. The results shown are typical of those obtained in at least three independent experiments. (D) rTMD1 blockage of the binding of CD14 to LPS. LPS was coated onto wells and incubated with indicated concentrations of rTMD1 and CD14. The binding of CD14 to LPS was detected using CD14 antibody (M-305, Santa Cruz Biotechnology). Values are the mean ± SD (n=4), ***P* < 0.01 and ****P* < 0.001 compared with the group that received only CD14. Similar results were obtained in three independent experiments.

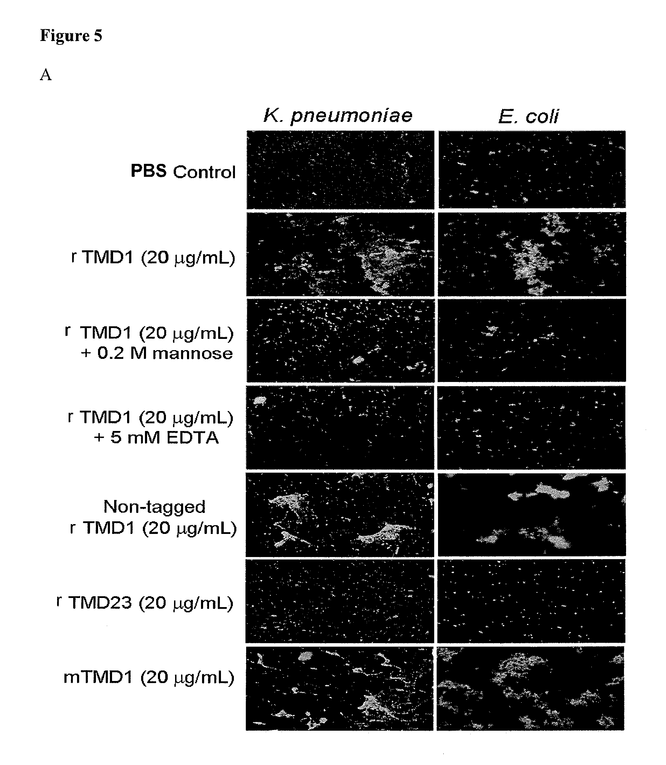

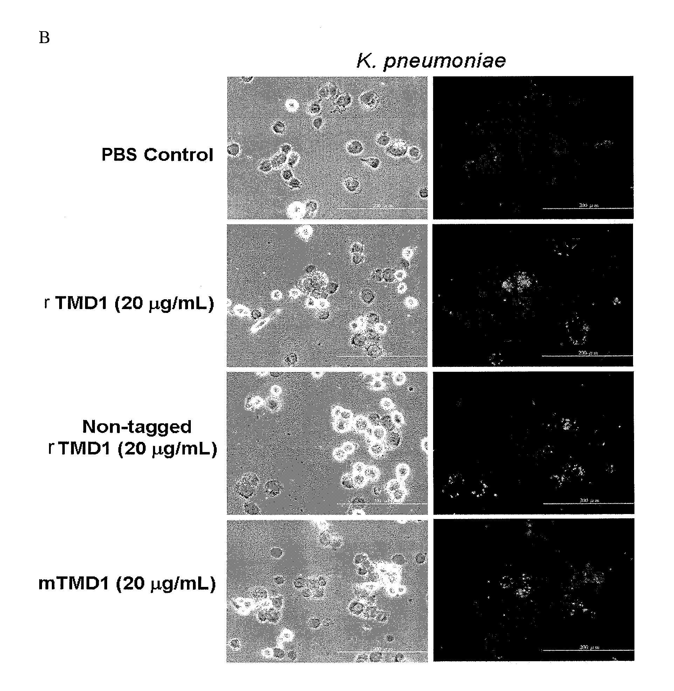

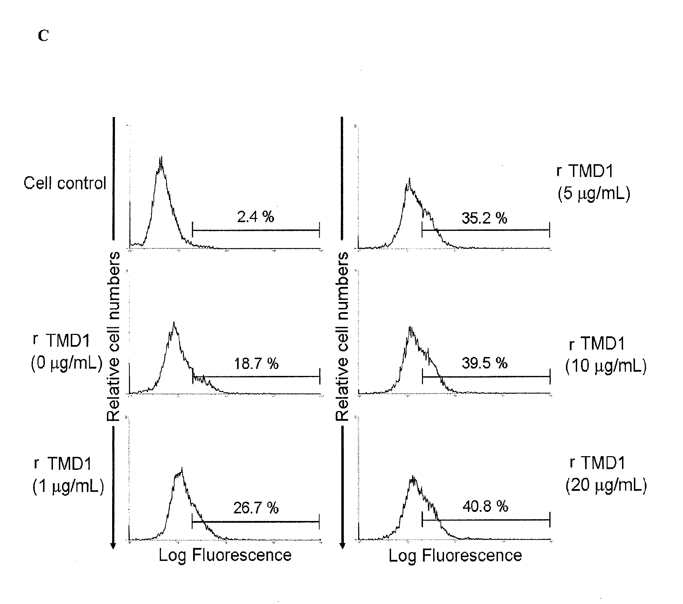

**Figure 5** shows the effects of rTMD1 on bacterial agglutination and phagocytosis by THP-1 cells. (A) FITC-labeled *K. pneumoniae* and *E. coli* DH5α were incubated in buffer containing 5 mM CaCl₂ and in the absence or presence of rTMD1 without or with 0.2 M mannose and 5 mM EDTA. The fluorescently-labeled bacteria were observed using a fluorescence microscope to evaluate bacterial agglutination. Non-tagged rTMD1 was prepared by incubation of rTMD1 with enterokinase and purified as described in Document S1. Mammalian expressed rTMD1 and an internal control with tagged rTMD23 were also included. Photomicrographs are representative of three independent experiments. (B) Representative photomicrograph showing the effect of rTMD1 on bacterial phagocytosis. Differentiated THP-1 cells were incubated with rTMD1-pretreated FITC-labeled *K. pneumoniae*. The left panel shows the bright field image and the right panel shows the fluorescence photomicrographs (scale bars represent 200 µm). (C) FACS analysis of the samples from (B). The graphs depict the level of FITC fluorescence (x-axis) vs. the relative cell numbers (y-axis). These graphs represent the results from three independent experiments.

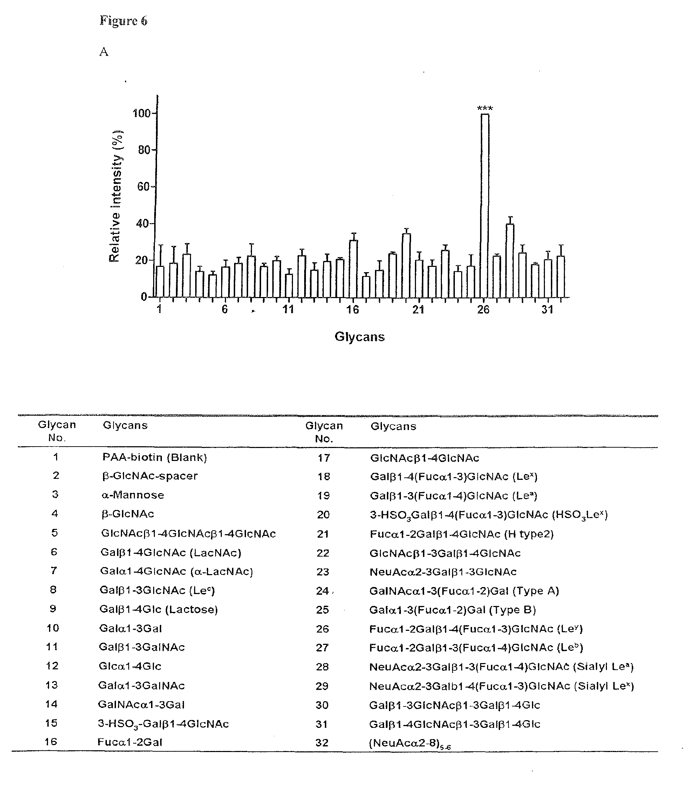

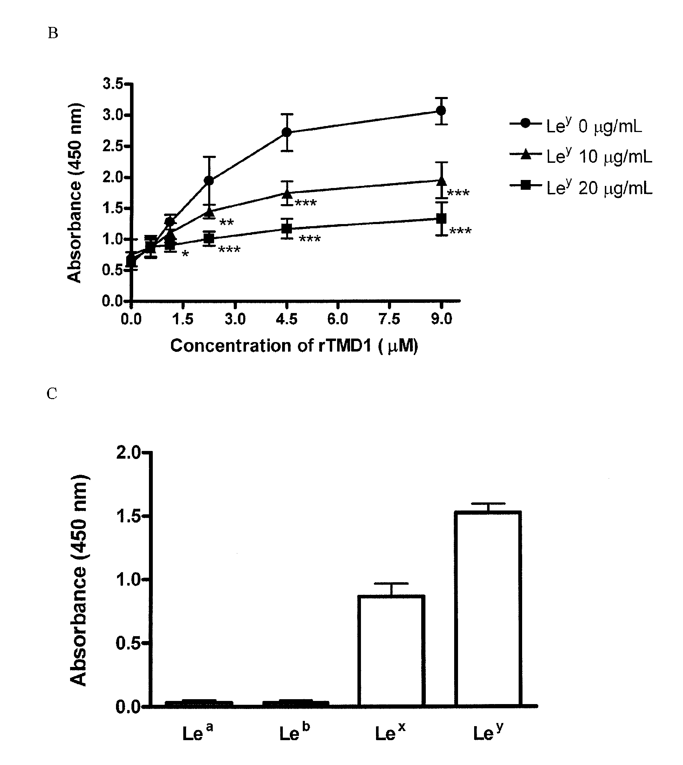

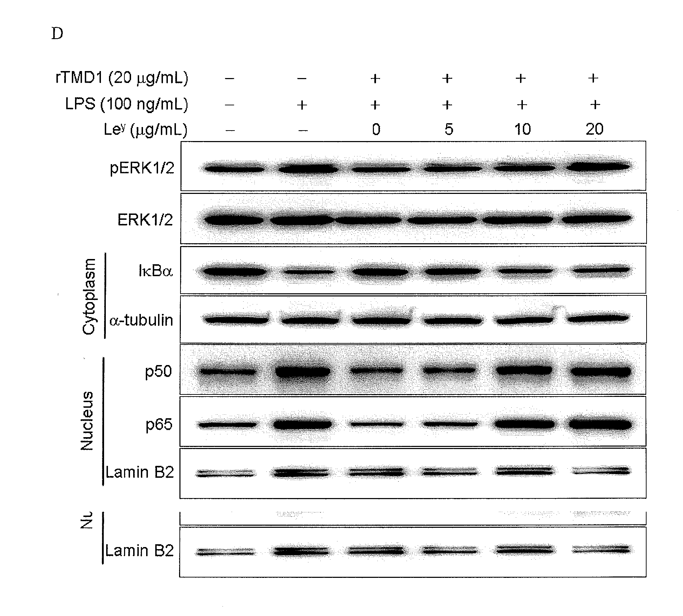

**Figure 6** shows the analysis of rTMD1 ligand by AlphaScreen, inhibitory effect of Le^{y} antigen on the binding of rTMD1 with LPS, and analysis of Lewis antigens in *E. coli* LPS O111:B4. (A) AlphaScreen assay results. Values are the mean ± SD (n=4). Sugar binding specificity of mammalian expressed rTMD1 is indicated by relative intensities (with reference to the highest absorbance unit). The sugar identities are designated by numbers as listed. ****P* < 0.001 compared with the blank. The results were obtained from the average of four independent assays. Similar result was obtained using *Pichia*-expressed rTMD1. (B) Effect of Le^{y} antigen on the binding of rTMD1 with LPS. LPS was coated onto wells and various concentrations of Le^{y} and rTMD1 were added to each well. The binding of rTMD1 is expressed as the mean ± SD (n=4), **P* < 0.05, ***P* < 0.01, and ****P* < 0.001 compared with the rTMD1 only group. Similar results were obtained in two independent experiments. (C) Analysis of Lewis antigens in *E. coli* LPS O111:B4. LPS (5 µg/well) was coated onto wells of high-binding microtiter plate. Le^{a}, Le^{b}, Le^{x}, and Le^{y} antibodies (5 µg/mL) were added to each well in binding buffer and incubated at 37°C for 2 hours followed by peroxidase-labeled secondary antibodies for 2 hours. The binding of Lewis antibodies to LPS was detected by measuring absorbance at 450 nm. Values are the mean ± SD (n=4). Similar results were obtained in two independent experiments. (D) Effect of Le^{y} on the blocking effect of rTMD1 on LPS-induced signaling pathways. rTMD1 (20 µg /mL) and various concentrations of Le^{y} were pre-incubated with LPS (100 ng/mL) before adding to cells. After incubation for 30 minutes, Western blot was used to assay LPS-induced ERK1/2 phosphorylation, degradation of IκBα in cytoplasmic fractions, and nuclear translocation of NF-κB p50 and p65 in nuclear fractions. Similar results were obtained in two independent experiments.

### DETAILED DESCRIPTION OF THE INVENTION

The present invention provides a use of a composition comprising N-terminal lectin-like domain of thrombomodulin (TMD1), or analogues thereof in the preparation of a medicament for treatment of a disease resulting from Lewis Y antigen over-expression. The Lewis Y antigen set forth is expressing in tumor cells, Gram-bacteria or LPS. In the embodiment, the tumor cells are epithelial-derived tumor, such as breast, pancreas, ovary, colon, gastric, and lung cancer. The use mentioned also prevents and treats tumor over-expressing Lewis Y antigen.

In this present invention, it is discovered that TMD1 binds to the LPS, inducing agglutination and enhancing bacteria phagocytosis by macrophages. It is shown that TMD1 functions as an anti-inflammatory factor in the early phase of systemic inflammation in sepsis induced by Gram-negative bacteria. Furthermore, it is demonstrated that TMD1 specifically interacts with bacteria carrying smooth-type LPS such as *Klebsiella pneumoniae (K. pneumoniae*), an important Gram-negative pathogen, and attenuates LPS- and *K. pneumoniae*-induced inflammatory responses and lethality by binding to LPS, blocking the downstream signal transduction. Thus, (TMD1 may have an important protective function in neutralization of LPS, of value therapeutically in the treatment of septic shock syndrome and other inflammatory disease states. The biological function of TM in macrophages is also proposed.

The current invention demonstrates that TM protects against Gram-negative bacteria-mediated sepsis. The N-terminal domain of thrombomodulin (TMD1) binds to the Lewis Y antigen and LPS, inhibiting LPS-induced inflammatory responses. Moreover, TMD1 causes agglutination of *Escherichia coli* (*E. coli* and *K. pneumoniae* and enhances the macrophage phagocytosis of these Gram-negative bacteria. Administration of TMD1 protects the host by suppressing inflammatory responses in sepsis induced by LPS and Gram-negative bacteria. Therefore, recombinant TMD1 may be valuable in treatment of severe inflammation in sepsis, especially in infection caused by Gram-negative bacteria.

In the preferred embodiment, the inflammatory response is sepsis induced by LPS or Gram-negative bacteria. In a further preferred embodiment, the Gram-negativebacteria is *E. coli* or *K. pneumoniae*.

'Inflammation' as used herein means the local reaction to injury of living tissues, especially the local reaction of the small blood vessels, their contents, and their associated structures. The passage of blood constituents through the vessel walls (extravasation) into the tissues is the hallmark of inflammation. Generally, inflammation starts with an enhanced leukocyte adhesion to the endothelial wall and results in leukocyte extravasation into tissues or organs.

In fact, any noxious process that damages living tissue infection with bacteria, excessive heat, cold, mechanical injury such as crushing, acids, alkalis, irradiation, or infection with viruses can cause inflammation irrespective of the organ or tissue involved. It should be clear that diseases of animals and man classed as 'inflammatory diseases' comprising arthritis, skin inflammation, peritonitis, injury associated with ischemia/reperfusion (eg. heart, liver, kidney, brain), inflammatory pulmonary disorders (including for example, asthma, bronchitis, adult respiratory distress syndrome (ARDS), vasculitis, atherosclerosis, nephritis, skin wound healing, sepsis, and local and systemic infections.

The term ' Gram-negative bacteria ' as used herein are those that do not retain crystal violet dye in the Gram staining protocol. Gram-positive bacteria will retain the dark blue dye after an alcohol wash. In a Gram stain test, a counter stain is added after the crystal violet, which colors all Gram-negative bacteria a red or pink color. The test itself is useful in classifying two distinctly different types of bacteria based on structural differences in their cell walls. Many species of Gram-negative bacteria are pathogenic, meaning they can cause disease in a host organism. This pathogenic capability is usually associated with certain components of Gram-negative cell walls, in particular the lipopolysaccharide (also known as LPS or endotoxin) layer.

The term ' Agglutination ' as used herein is the clumping of particles. This occurs in biology in three main examples: 1. The clumping of cells such as bacteria or red blood cells, in the presence of an antibody. The antibody or other molecule binds with multiple particles, and joining them. 2. The coalescing of small particles that are suspended in solution; these larger masses are then (usually) precipitated. 3. An allergic reaction type occurrence where cells become more compacted together to prevent foreign materials entering them. This is usually the result of an antigen in the vicinity of the cells. The agglutination of bacteria of the present invention facilitates phagocytosis of the bacteria by macrophages.

In the preferred embodiment, the disease is sepsis induced by LPS or Gram-negative bacteria. The disease or disorder is prevented or treated by binding N-terminal lectin-like domain of thrombomodulin (TMD1) to LPS or Gram-negative bacteria to induce agglutination or opsonization of bacteria. Thus, the disease or disorder is prevented or treated by reducing LPS-induced production of the inflammatory mediators TNF-α or NO in macrophages and by suppressing signal pathways involved in LPS-induced inflammatory responses.

In the further preferred embodiment, the disease or disorder is prevented or treated by interaction of TMD1. TMD1 has a function as a natural opsonic moiety for innate immunity against Gram-negative bacteria. The induction of opsonization is made by binding TMD1 to Lewis Y antigen in LPS or cell membrane, and the induction of agglutination of bacteria facilitates phagocytosis of the bacteria by macrophages. Those responses alleviate PMN infiltration in lungs or preserves kidney function. The signal pathways involved in LPS-induced inflammatory responses includes phosphorylation of ERK and P38, degradation of IκB and nuclear translocation of NF-κB and increasing expression of inducible nitric oxide synthase (iNOS) and TNF-α. Besides, the disease or disorder is prevented by interaction TMD1 with high mobility group B 1(HMGB1).

The present invention also provides a method of detecting cancer cells, Gram-negative bacteria or LPS which over-express the Lewis Y antigen in a sample from a subject, said method comprising the step: contacting a sample with a composition comprising a labeled N-terminal lectin-like domain of thrombomodulin (TMD1), or labeled analogues. In the preferred embodiment, the cancer cells which express the Lewis Y antigen is breast, pancreas, ovary, colon, gastric, or lung cancer. The sample set forth is derived from blood, tissue, body fluid, cell, or excrement from the subject. In the embodiment of this invention, the subject is mammal. In the preferred embodiment of this invention, the subject is human. The labeled marker in the TMD1 or its analogues is detectable marker, such as fluorescent molecules, radioactive molecules, chromogenic molecules, biotin, acridinium ester and acridinium-9-carboxamide.

The term ' subject' as used herein is any animal who receives medical attention, care, or treatment. In particularly, the animal is a human. The human is the most often ill or injured and in need of treatment by a physician or other medical professional.

The term ' analogues ' as used herein are compounds in which one or more individual atoms have been replaced, either with a different atom, or with a different functional group. Another use of the term in biochemistry refers to a substance which is similar in structure to another substance. The analogues of TMD1 used herein includes lectin domain of TM from different animals including murine but not limited to and the modification known to the art of people with knowledge of the field such as point mutation and chemical modification such as PEGylation and glycosylation

The term ' innate immunity ' as used herein comprises the cells and mechanisms that defend the host from infection by other organisms, in a non-specific manner. This means that the cells of the innate system recognize, and respond to, pathogens in a generic way, but unlike the adaptive immune system, it does not confer long-lasting or protective immunity to the host. Innate immune systems provide immediate defense against infection, and are found in all classes of plant and animal life.

The term ' opsonization ' as used herein is any molecule that acts as a binding enhancer for the process of phagocytosis, for example, by coating the negatively-charged molecules on the membrane. Both the membrane of a phagocytising cell, as well as its target, have a negative charge (Zeta-potential), making it difficult for the two cells to come close together. During the process of opsonization, antigens are bound by antibody and/or complement molecules. Phagocytic cells express receptors that bind opsonin molecules. With the antigen coated in these molecules, binding of the antigen to the phagocyte is greatly enhanced. Most phagocytic binding cannot occur without opsonization of the antigen. Furthermore, opsonization of the antigen and subsequent binding to an activated phagocyte will cause increased expression of complement receptors on neighboring phagocytes. Examples of opsonin molecules include the IgG and IgA antibodies and the C3b, C4b, and iC3b components of the complement system.

The present invention further provides a kit of detecting Lewis Y antigen over-expressed in tumor cells, Gram-negative bacteria or LPS comprising a labeled N-terminal lectin-like domain of thrombomodulin (TMD1), or labeled analogues thereof.

### EXAMPLES

### Material and Methods

### 1. Preparation of recombinant TMD (rTMD) proteins using both Pichia and mammalian protein expression systems.

The pPICZαA and pCR3-EK vectors were used for expression and secretion of human rTMD proteins containing 6xHis tag and c-Myc epitope for purification and for protein detection in the *Pichia pastoris* and human embryonic kidney 293 (HEK293) mammalian protein expression systems. Briefly, DNA fragments coding for TMD1 (residues Ala¹-Ala¹⁵⁵), TMD2/EGF123 (residues Ala²²⁴-Glu³⁴⁶), and TMD23 (residues Ala²²⁴-Ser⁴⁹⁷) were obtained by a PCR of human umbilical vein endothelial cell cDNA using specific primers. The primer sequences used for construction were as follows: TMD1 (TMD1-sense: SEQ ID NO: 2 and TMD1-antisense: SEQ ID NO: 3), TMD23 (TMD2-sense: SEQ ID NO: 4 and TMD3-antisense: SEQ ID NO: 5), and TMD2/EGF123 (TMD2-sense and TMD2/EGF3-antisense: SEQ ID NO: 6). An enterokinase cutting site between the rTMD sequence and His/c-Myc tag allowed subsequent removal of the tag sequence. Yeast fermentation medium or HEK293 cell conditioned medium containing expressed rTMD proteins was applied to a nickel-chelating Sepharose column (Amersham Pharmacia Biotech., Piscataway, NJ), and rTMD-containing fractions were eluted in an imidazole gradient. The purified rTMD proteins were examined by SDS-PAGE and Western blotting, and were verified by protein identification with mass spectrometry analysis. The N-terminal amino acid sequence of rTMD proteins was also determined by Edman degradation with a model 477A sequencer (Applied Biosystems, Foster City, CA). The molecular masses of rTMD proteins containing fusion peptides were predicted using the ExPAsy website for calculating protein concentration (rTMD 1: 22,902 Da, rTMD2/EGF123: 16,473 Da, and rTMD23: 32,823 Da). A non-tagged rTMD1 was prepared by incubation of 1mg rTMD1 with 5 ng enterokinase (New England Biolab, Beverly, MA) at 4°C for 16 hours. After incubation, the sample was applied to a nickel-chelating Sepharose column and the non-bound fraction containing the non-tagged rTMD1 was collected. The sample was further treated with immobilized soy bean trypsin inhibitor-Sepharose gel to adsorb the residual enterokinase. Each sample was analyzed before and after enterokinase treatment by SDS-PAGE and Western blot, and non-tagged rTMD1 having a molecular mass 2000 Da less than the original tagged rTMD1 was obtained.

### 2. Cells and cell culture.

Human leukemia monocytic THP-1 cells and murine macrophage cell line RAW 264.7 were obtained from the American Type Culture Collection. THP-1 cells were grown in suspension in RPMI-1640 supplemented with 10 mM HEPES, 1 mM sodium pyruvate, 0.1% β-mercaptoethanol, 100 U/ml penicillin, 100 g/ml streptomycin, and 10% fetal bovine serum (FBS). RAW 264.7 cells were grown as an adhered layer in Dulbecco's modified Eagle's medium with 4 mM L-glutamine adjusted to contain 1.5 g/l sodium bicarbonate and 4.5 g/l glucose, 100 U/ml penicillin, 100 g/ml streptomycin, and 10% FBS under a humidified 5% CO₂ atmosphere at 37°C. For differentiation, THP-1 cells were plated in medium containing 10 nM PMA and allowed to adhere for 18 h.

### 3. Inflammatory mediator assays.

TNF-α in conditioned medium or mouse serum was measured by an enzyme-linked immunosorbent assay (ELISA) (R&D Systems, Minneapolis, MN). Accumulation of nitrite in the medium or mouse serum was determined by a colorimetric assay using Griess reagent (Sigma-Aldrich). Blood urea nitrogen (BUN) and creatinine were measured with a Roche D&P modular (Roche Diagnostic Systems, Branchburg, NJ). Assays were performed according to the manufacturer's protocol.

### 4. Western blotting.

rTMD1 (0-50 µg/mL, equals 0-2.18 nmole/mL) pre-incubated without or with *Escherichia coli* (*E. coli*) LPS (LPS 0111:B4, Sigma-Aldrich) in the absence or presence of Lewis Y (Le^{y}; Dextra Laboratories, Reading, UK) for 30 minutes was used to stimulate THP-1 or RAW 264.7 cells. Incubation period included 15 minutes for pIκB and IκB (B-9 and C-21, Santa Cruz Biotechnology, Santa Cruz, CA, unless otherwise noted), 20 minutes for p38 (C-20) and pp38 (D-8), 30 minutes for ERK1/2 (K-23), pERKI/2 (E-4), lamin B2 (E-3, Zymed Laboratories, San Francisco, CA), NF-κB p50 (C-19) and NF-κB p65 nuclear translocation (C-20), and 24 hours for iNOS expression using a specific antibody (N-20).

### 5. Animals and systemic sepsis models.

Male FVB mice (8-10 weeks old) were used to model sepsis in vivo. The Institutional Animal Care and Use Committee of the National Cheng Kung University approved procedures. In the chronic endotoxemia model, various concentrations of rTMD1 or rTMD proteins in equi-molar amounts were administrated by tail intravenous (i.v.) injection and LPS (20 mg/kg) or *K. pneumoniae* (5×10² CFU/mouse, BCRC) was administrated by intraperitoneal (i.p.) injection. After 6 or 12 hours, mice were sacrificed by pentobarbital anesthesia (50 mg/kg; i.p.). Sera were collected and assayed for TNF-α , NO, BUN, and creatinine, and lung and kidney tissues were removed, fixed with formalin, and embedded in paraffin for histochemical examination. In the lethal sepsis model, rTMD1 (2 mg/kg, equals 87.3 nmole/kg) was i.v.-administrated at 0, 6, 12, and 24 hours after i.p. administration of LPS (40 mg/kg). The half-life of rTMD1 in the circulation was determined by i.v. injection of rTMD1 (10 mg/kg, equals 436.64 nmole/kg) and serum samples were collected at various time intervals. The levels of rTMD1 in the collected sera were determined by a sandwich ELISA which employed anti-c-Myc monoclonal antibody and TM-H300 as capture and detection antibodies (both from Santa Cruz Biotechnology), respectively. For the lethal bacteremia model, rTMD1 (10 mg/kg) was administrated prior to i.p. injection of *K. pneumoniae* (5×10³ CFU/mouse). Mortality was monitored every 6-12 hours until all mice in either experimental group died. Experimental procedure of determining LPS half-life is as following. LPS (20 mg/kg) was i.p.-administrated to male FVB mice without or with rTMD1 (10 mg/kg; i.v.). Serum samples were collected at various time intervals and the amount of LPS was determined by Limulus amebocyte lysate test (Associates of Cape Cod, E. Falmouth, MA). A further experiment was performed to determine whether rTMD1 affects bacterial clearance. *K. pneumoniae* (5×10² CFU/mouse) was i.p.-administrated to male FVB mice without or with rTMD1 (10 mg/kg; i.v.), and blood samples were collected at various time intervals. Outgrowth of *K. pneumoniae* was quantified by plating serial dilutions of blood samples on blood agar plates and enumerating colonies after overnight incubation at 37°C.

### 6. K. pneumoniae and LPS binding assays.

*K. pneumoniae* (3×10⁵ CFU/well), *E. coli* O111:B4 smooth-type LPS (5 µg/well), or bovine serum albumin (BSA, 5 µg/well) in 100 µL of bicarbonate buffer (pH 9.6) were coated onto wells of high-binding microtiter plate (Corning Costar, Cambridge, MA). Nonspecific binding was blocked with binding buffer (20 mM Tris-HCl, pH 7.4, 0.15 M NaCl, 5 mM CaCl₂) containing 50 mg/mL BSA. Various concentrations of rTMD proteins without or with CD14 (10 µg/mL; R&D Systems) in binding buffer containing 1 mg/mL BSA were added to wells and incubated for 2 hours. In some experiments, 0.2 M mannose, 5 mM EDTA, or Le^{y} were included in the binding buffer to compete with LPS and rTMD1 binding. Antibody against the c-Myc epitope of the rTMD proteins was added to wells and incubated for 2 hours, and peroxidase-labeled secondary antibody followed. Binding of rTMD proteins was detected by measuring absorbance at 450 nm.

### 7. Effect of rTMD1 on bacterial agglutination.

*K. pneumoniae* and *E. coli* DH5α (BCRC) were washed twice in 0.1 M sodium bicarbonate buffer (pH 9.6) and incubated with 0.1 mg/mL fluorescein isothiocyanate (FITC) at 37°C in the dark. Each of 20 µg/mL of rTMD1, non-tagged rTMD1, mammalian rTMD1, and recombinant TM domains 2 and 3 (rTMD23) was co-incubated with FITC-labeled bacteria (1×10⁵ CFU/mL) in buffer (20 mM Tris, pH 7.4, 0.15 M NaCl, 5 mM CaCl₂, 1 mg/mL BSA) with or without 0.2 M mannose or 5 mM EDTA. Samples were individually placed on a glass slide and fluorescently-labeled bacteria were observed microscopically to evaluate agglutination.

### 8. THP-1 phagocytosis assay.

FITC-labeled *K. pneumoniae* (1×10⁵ CFU/well) were pre-incubated with rTMD1 at 37°C for 30 minutes before adding to differentiated THP-1 cells. After incubation for 2 hours the THP-1 cells were washed twice with ice-cold PBS prior to fluorescence microscopy, or were further trypsinized, resuspended in PBS, and analyzed in a fluorescence-activated cell sorter (FACS; Becton Dickinson, San Jose, CA) to measure THP-1 cell intracellular fluorescence. Green fluorescence data from 10,000 events (cells) per condition collected at 530 nm on a log scale were analyzed with CellQuest™ software (Becton Dickinson).

### 9. rTMD1 ligand analysis.

Biotin-polyacrylamide (Biotin-PAA)-sugars (GlycoTech, Rockville, MA), rTMD1, rabbit anti-mouse IgG antibody (Zymed), mouse anti-6× His tag antibody (Abcam, Cambridge, UK), and streptavidin-coated donor and protein A conjugated acceptor beads (PerkinElmer, Boston, MA) were diluted with assay buffer containing 25 mM Tris (pH 7.0), 25 mM CaCl₂, and 1 mg/mL BSA to appropriate concentrations. The anti-6x His tag antibody, rabbit anti-mouse IgG antibody, and acceptor beads were incubated with assay buffer for 1 hour at 25°C before use (as the acceptor mixture). Biotin-PAA-sugars, rTMD1, and donor beads were separately added to wells of ProxiPlate-384 assay plates (PerkinElmer) and incubated at 25°C for 1 hour. An aliquot of the acceptor mixture was then added to the wells and incubated at 25°C for another 2 hours. The results were read on a PerkinElmer Envision instrument using the AlphaScreen program. All the procedures and incubations were carried out in the dark.

### 10. Analysis of Lewis antigens in E. coli LPS O111:B4.

*E. coli* O111:B4 LPS (5 µg/well) was coated onto wells of a high-binding microtiter plate. After the nonspecific binding blocked with binding buffer containing 50 mg/mL BSA, 5 µg/mL of various kinds of antibodies (Abcam) against Lewis a (Le^{a}), Lewis b (Le^{b}), Lewis X (Le^{x}), and Le^{y} were added to wells with binding buffer containing 1 mg/mL BSA and incubated at 37°C for 2 hours prior to incubation with goat anti-mouse horseradish peroxidase-conjugated IgG or IgM at 37°C for 2 hours. The peroxidase reaction was performed using 3,3',5,5'-tetramethylbenzidine as a substrate and was stopped by 2N H₂SO₄. The products were detected by measuring absorbance at 450 nm.

### 11. Statistical analyses.

Survival data were analyzed by log-rank test. Data expressed as the mean ± standard deviation (SD). Statistical significance was analyzed by unpaired Student's *t* test. Differences between more than two groups were compared by one-way analysis of variance or two-way analysis of variance and following Bonferroni's post hoc test, with P < 0.05 considered statistically significant.

### EXAMPLE 1: rTMD1 treatment reduces LPS-induced inflammatory mediator production in macrophages.

To test whether TMD1 has anti-inflammatory property, rTMD proteins were prepared by using both *Pichia pastoris* and mammalian protein expression systems. The rTMD1 proteins obtained from both systems had similar molecular mass with glycosylation modification, which was about 35 kDa and as assayed by silver staining and Western blotting. The anti-inflammatory effect of rTMD proteins in RAW 264.7 and THP-1 cells stimulated with LPS was tested first. *Pichia*-expressed rTMD1 but not recombinant TMD2 containing EGF like domain 1, 2, 3 (rTMD2/EGF123) dose-dependently inhibited TNF-α production in RAW 264.7 cells stimulated with LPS (Figure 1A); similar results were obtained in THP-1 cells (data not shown). Likewise, only rTMD1 could effectively inhibit NO production in RAW 264.7 cells (Figure 1B); similar results were obtained using mammalian-expressed rTMD1 (Figure 1A-B). To test whether the reduction of LPS-induced inflammatory mediator by rTMD1 results from LPS-rTMD1 binding, various concentrations of LPS and rTMD1 were used to test the interaction. In the absence of rTMD1, LPS dose-dependently increased TNF-α production (Figure 1C) and rTMD1 dose-dependently inhibited LPS-induced TNF-α production (Figure 1C), which are consistent with the hypothesis that LPS-rTMD1 binding results in the reduction effect of rTMD1 on the inflammatory mediator production by the cells.

### EXAMPLE 2: rTMD1 blocks the LPS-induced signaling pathways.

Phosphorylation and degradation of IκBα occurred in RAW 264.7 cells stimulated with LPS (100 ng/mL). The effect was totally reversed by rTMD1 (50 µg/mL, equals 2.18 nmole/mL) (Figure 1D). The LPS-induced nuclear translocation of NF-κB was also inhibited in a dose-dependent fashion by rTMD1 (Figure 1E). LPS-induced phosphorylation of ERK1/2 and p38 was also inhibited by rTMD1 (Figure 1F). Similar effects of rTMD1 on activation of signal transduction pathway were observed in THP-1 cells (data not shown). iNOS induction in the RAW 264.7 cells by LPS was also inhibited by rTMD1 (Figure 1 G).

### EXAMPLE 3: rTMD1 reduces cytokine release, attenuates lung and renal injury, improves survival in experimental sepsis, and enhances bacterial LPS clearance.

Since rTMD1 could inhibit LPS-induced inflammatory mediator productions and signaling pathways, rTMD1 might function as a therapeutic agent to reduce the inflammatory response and lethality induced by LPS in vivo. TNF-α and NO levels were increased in mice 6 hours after i.p. administration of 20 mg/kg LPS, relative to control mice. Mice receiving an i.v. injection of rTMD1 (1-5 mg/kg, equals 43.66-218.3 nmole/kg) had significantly decreasing levels of TNF-α and NO (Figure 2A-B). PMNs infiltration was evident in lung sections in LPS-treated mice 6 hours after administration of LPS (Figure 2C-D). rTMD1 injection significantly inhibited pulmonary accumulation of PMNs 6 hours after LPS administration (Figure 2C-D). However, rTMD2/EGF123 had no significant effect on LPS-induced inflammatory responses and could serve as a negative control of yeast recombinant protein (Figure 2C-D). Similarly, LPS caused severe glomerular injury of the kidney. The extent of glomerulonephritis was significantly reduced in rTMD1-treated mice (Figure 2E). The increase of BUN and serum creatinine 12 hours after administration of LPS was consistent with the functional failure of the kidney. Mice treated with rTMD1 and then challenged with LPS had markedly reduced BUN and creatinine levels comparing with LPS-treated mice (Figure 2F-G). To study whether this effect protects against lethality, the mice were treated with four i.v.-administered doses of rTMD1 (2 mg/kg) or PBS and observed the mice until all mice in either experimental group died. rTMD1 treatment possessed significant protection against lethality and improved survival during endotoxemia (rTMDI-treated group survival, 100%; PBS-treated group survival, 10%, 1 day after LPS challenge; and rTMD1-treated group survival, 60%; PBS-treated group survival, 0%, 4 days after LPS challenge) (Figure 2H), suggesting that rTMD1 may have therapeutic potential. The injected rTMD1 was detectable within 12 hours after each i.v. injection with a 3-4 hours half-life as assayed by ELISA (Figure 2I). The injected LPS (20 mg/kg) reached a maximum level 2 hours after i.p.-administration and was cleared from the circulation with an approximate 6-8 hours half-life (Figure 2J). rTMD1 administration significantly enhanced LPS clearance (Figure 2J). In addition, a rabbit polyclonal antibody against rTMD1 (TMD1 Ab) was prepared and characterized. Pre-treatment of rTMD1 with TMD1 Ab reversed the rTMD1's effect on the suppression of LPS-induced TNF-α release, suggesting that the anti-inflammatory effect of rTMD1 on the inflammatory mediator production is rTMD1-specific.

### EXAMPLE 4: rTMD1 reduces K. pneumoniae-induced inflammatory responses and lethality, and enhances bacterial clearance.

To test whether rTMD1 protects against lethality induced by Gram-negative bacteria, systemic sepsis was induced in mice by injecting i.p. with *K. pneumoniae* and treating with rTMD1 (Figure 3A-C). TNF-α and NO levels were increased within 12 hours in mice receiving *K. pneumoniae* (5×10² CFU). rTMD1 treatment (5-25 mg/kg; i.v.) effectively reduced the TNF-α and NO production (Figure 3A-B). For the survival experiment, all mice received *K. pneumoniae* (5×10³ CFU) died within 18 hours, whereas 50% of the mice received a single i.v. dose of rTMD1 (10 mg/kg) survived more than 24 hours (Figure 3C). To explore whether the effect of *K. pneumoniae*-induced mortality results from rTMD1 promoted bacterial clearance, the amount of viable bacteria in the blood was determined. FVB mice were each i.p. injected with *K. pneumoniae* (5×10² CFU) without or with rTMD1 treatment (10 mg/kg; i.v.). rTMD1 significantly enhanced *K. pneumoniae* clearance in circulation at 12, 24, and 36 hours after injection (Figure 3D). To demonstrate that rTMD1 has therapeutic potential, rTMD1 was infused after bacterial infection. In time course experiments, rTMD1 (10 mg/kg) was i.v.-administrated immediately (within a minute), 30, or 60 minutes after i.p. administration of *K. pneumoniae*. The result showed that rTMD1 could effectively suppress *K. pneumoniae*-induced TNF-α release (Figure 3E) and enhance *K. pneumoniae* clearance in circulation at 12, 24, and 36 hours after infection even at 30 or 60 minutes post-treatment of rTMD1 (Figure 3F). Mice infected with *K. pneumoniae* in the absence of rTMD1 started to die after 42 hours; however, the survival was improved in all rTMD1-treated groups.

### EXAMPLE 5: Direct binding of rTMD1 with Gram-negative bacteria and LPS, and direct binding of membrane-bound TM with LPS.

To test whether TMD1 is capable of specific binding to Gram-negative bacteria and LPS, rTMD1 and rTMD23 were used for binding with *K. pneumoniae*, LPS, or BSA. rTMD1 but not rTMD23 could specifically bind to *K. pneumoniae* (Figure 4A). Since rTMD1 could bind to Gram-negative bacteria, LPS of Gram-negative bacteria was assumed a potential candidate ligand of rTMD1. Appropriately, binding of rTMD1 and rTMD23 to LPS or BSA was measured. rTMD1 but not rTMD23 specifically bound to LPS (Figure 4B) and binding was inhibited by mannose and EDTA, suggesting that the TMD1 carbohydrate recognition domain interacted with LPS carbohydrate in a Ca²⁺-dependent manner (Figure 4C). From Figure 4B, the apparent Kd estimated from the 50% saturation of the binding of LPS with rTMD1 was about 1.6×10⁻⁶ mole/L. Also, LPS binds rTMD1 in a concentration-dependent manner with Kd ranged from 8.24×10⁻⁶ mole/L to 9.01×10⁻⁸ mole/L using surface plasmon resonance (SPR) assay. It was conceivable that the binding of rTMD1 to LPS might block the interaction of LPS with LPS-binding molecules, which blocks the LPS-induced inflammatory mediator productions and signaling pathways. As shown in Figure 4D, the binding of CD 14 to LPS was inhibited by rTMD1 in a dose-dependent manner, but the binding of LPS binding protein to LPS was not inhibited by rTMD1 (data not shown). To explore whether the endogenous TMD1 can specifically interact with LPS as rTMD1does, endogenous membrane-bound TM¹⁴ and overexpressed full-length or lectin-like domain-deleted membrane-bound TM^{17,29} were designed to test LPS binding capacity. The results showed that membrane-bound TM expressed on THP-1 cells could bind biotinylated LPS. In addition, full-length but not lectin-like domain-deleted membrane-bound TM could bind biotinylated LPS. These results suggest that membrane-anchored TM could bind LPS via TM's N-terminal lectin-like domain.

### EXAMPLE 6: rTMD1 induces agglutination of Gram-negative bacteria and enhances bacterial phagocytosis in THP-1 cells.

Since TMD1 mediates cell-cell adhesion by binding to carbohydrate ligands ¹⁷ and rTMD1 functions in clearance of LPS and *K. pneumoniae* (Figures 2J and 3D, respectively), it was postulated that rTMD1 may participate in binding and phagocytosis of bacteria if the carbohydrate moieties on the surface of the bacteria consist of ligands of TM. rTMD1 binding specificity was assayed by an agglutination reaction with FITC-labeled Gram-negative (*K. pneumoniae* and *E. coli*) bacteria. rTMD1 (20 µg/mL) induced marked agglutination of *K. pneumoniae* and *E. coli* DH5α (Figure 5A). Moreover, the agglutination activity was Ca²⁺-dependent and was attenuated by mannose and EDTA (Figure 5A). To rule out the possible interference effect of His/c-Myc tag in rTMD proteins, it was prepared a non-tagged rTMD1 to perform the test. A non-tagged rTMD1 showed the same effect as rTMD1 while tagged rTMD23 failed to produce agglutination; similar result was obtained by using mammalian-expressed rTMD1 (Figure 5A). The effect of rTMD1 on the phagocytosis of FITC-labeled *K. pneumoniae* by activated THP-1 cells was assayed. rTMD1 markedly increased the amount of bacterial phagocytosis by THP-1 cells (Figure 5B-C). Therefore, rTMD1 not only can interfere with the bacteria-induced inflammatory reaction, but also induce agglutination of bacteria and promote phagocytosis by macrophages.

### EXAMPLE 7: Identification of ligand specificity of rTMD1 and no competing effect of Le^{y} on HMGB1-rTMD1 binding.

To identify the ligand specificity of TMD1, a panel of carbohydrate ligands (Figure 6A) was tested for rTMD1 affinity using the AlphaScreen method. The result showed that Le^{y} antigen was a specific ligand for either mammalian- or *Pichia*-expressed rTMD1 (Figure 6A). Le^{y} specifically inhibited binding of rTMD1 with LPS in a dose-dependent manner (Figure 6B), indicating that the carbohydrate binding site of rTMD1 was involved in the interaction with LPS. ELISA demonstrated that *E. coli* O111:B4 LPS contained Le^{x} and Le^{y} antigens rather than Le^{a} and Le^{b} antigens (Figure 6C), consistent with the binding specificity of rTMD1. Furthermore, Le^{y} could dose-dependently neutralize the blocking effects of rTMD1 on LPS-induced ERK1/2 phosphorylation, IκBα degradation, and NF-κB p50 and p65 nuclear translocation (Figure 6D). An experiment to observe the effect of Le^{y} on the binding of HMGB1 with rTMD1 was performed for examining whether LPS/Le^{y}-rTMD1 interactions interfere with HMGB1-rTMD1 interactions and thus the binding sites of LPS/Le^{y} and HMGB1 overlap on TMD1. The result showed that Le^{y} could not abrogate the HMGB1 and rTMD1 interaction, suggesting that structural domain of TMD1 mediating HMGB1 interaction does not overlap with LPS/Le^{y}.

| Glycan No. | Glycans | Glycan No. | Glycans |
|---|---|---|---|
| | | | |
| 1 | PAA-biotin (Blank) | 17 | GlcNAcβ-4GlcNAc |
| 2 | β-GlcNAc-spacer | 18 | Galβ1-4(Fucα1-3)GlcNAc (Le^{x}) |
| 3 | α-Mannose | 19 | Galβ1-3(Fucα1-4)GlcNAc (Le^{a}) |
| 4 | β-GlcNAc | 20 | 3-HSO₃Galβ1-4(Fucα1-3)GlcNAc (HSO₃Le^{x}) |
| 5 | GlcNAcβ1-4GlcNAcβ1-4GlcNAc | 21 | Fucα1-2Galβ1-4GlcNAc (H type2) |
| 6 | Galβ1-4GlcNAc (LacNAc) | 22 | GlcNAcβ1-3Galβ1-4GlcNAc |
| 7 | Galα1-4GlcNAc (α-LacNAc) | 23 | NeuAcα2-3Galβ1-3GlcNAc |
| 8 | Galβ1-3GlcNAc (Le^{c}) | 24 | GalNAcα1-3(Fucα1-2)Gal (Type A) |
| 9 | Galβ1-4Glc (Lactose) | 25 | Galα1-3(Fucα1-2)Gal (Type B) |
| 10 | Galα1-3Gal | 26 | Fucα1-2Galβ1-4(Fucα1-3)GlcNAc (Le^{y}) |
| 11 | Galβ1-3GalNAc | 27 | Fucα1-2Galβ1-3(Fucα1-4)GlcNAc (Le^{b}) |
| 12 | Glcα1-4Glc | 28 | NeuAcα2-3Galβ1-3(Fucα1-4)GlcNAc (Sialyl Le^{a}) |
| 13 | Galα1-3GalNAc | 29 | NeuAcα2-3Galb1-4(Fucα1-3)GlcNAc (Sialyl Le^{x}) |
| 14 | GalNAcα1-3Gal | 30 | Galβ1-3GlcNAcβ1-3Galβ1-4Glc |
| 15 | 3-HSO₃-Galβ1-4GlcNAc | 31 | Galβ1-4GlcNAcβ1-3Galβ1-4Glc |
| 16 | Fucα1-2Gal | 32 | (NeuAcα2-8)₅₋₆ |

## Claims

1. Use of a composition comprising N-terminal lectin-like domain of thrombomodulin (TMD1) of SEQ ID NO: 1, or analogues thereof in the preparation of a medicament for the treatment of a disease resulting from Lewis Y antigen over-expression.

2. The use of claim 1, wherein the Lewis Y antigen is expressing in tumor cells, Gram-negative bacteria or LPS.

3. The use of claim 1, wherein the disease is a tumor or an inflammatory disease.

4. The use of claim 2, wherein the tumor cells are epithelial-derived tumor cells.

5. The use of claim 4, wherein the epithelial-derived tumor is breast, pancreas, ovary, colon, gastric, or lung cancer.

6. The use of claim 3, wherein the inflammatory disease is induced by LPS or Gram-negative bacteria.

7. The use of claim 6, wherein the inflammatory disease is sepsis.

8. The use of claim 1, wherein the treatment or prevention is made by binding N-terminal lectin-like domain of thrombomodulin (TMD1) of SEQ ID NO: Ito Lewis Y antigen to induce agglutination, opsonization of bacteria or phagocytosis by macrophages.

9. The use of claim 8, wherein TMD1 has a function as a natural opsonic moiety for innate immunity against Gram-negative bacteria.

10. The use of claim 8, wherein the binding N-terminal lectin-like domain of thrombomodulin (TMD1) of SEQ ID NO: 1 to Lewis Y antigen results in reducing LPS-induced production of inflammatory mediators TNF-α or NO in macrophages, suppressing signal pathways involved in LPS-induced inflammatory responses, alleviating PMN infiltration in lung and preserving kidney function.

11. The use of claim 10, wherein the signal pathways involved in LPS-induced inflammatory responses include phosphorylation of ERK and P38, degradation of I_{κ}B and nuclear translocation of NF- _{κ}B and increasing expression of inducible nitric oxide synthase (iNOS).

12. A kit of detecting Lewis Y antigen over-expressed in tumor cells, Gram-negative bacteria or LPS comprising a labeled N-terminal lectin-like domain of thrombomodulin (TMD 1) of SEQ ID NO: 1, or labeled analogues thereof.

13. A method of detecting tumor cells, Gram-negative bacteria or LPS which over-expresses the Lewis Y antigen in a sample from a subject, said method comprising the step: contacting a sample with a composition comprising a labeled N-terminal lectin-like domain of thrombomodulin (TMD1) of SEQ ID NO: 1, or labeled analogues thereof.

14. The method of claim 13, wherein the tumor cells which express the Lewis Y antigen is breast, pancreas, ovary, colon, gastric, or lung cancer cells.

15. The method of claim 13, wherein the sample is blood, tissue, body fluid, cell, or excrement from the subject.
